# FASCICULE DE BREVET EUROPEEN

(11) **EP 3 414 565 B1**
(45) Date de publication et mention de la délivrance du brevet: **27.10.2021**
(21) Numéro de dépôt: 17701726.6
(22) Date de dépôt: 30.01.2017
(51) Int. Cl.: G01N 33/28

(54) **APPAREIL D'ANALYSE DE LA SENSIBILITÉ À LA FORMATION DE DÉPÔT DANS UN CARBURANT, NOTAMMENT DANS UN CARBURANT UTILISÉ EN AÉRONAUTIQUE**
VORRICHTUNG ZUR ANALYSE DER NEIGUNG ZUR BILDUNG VON ABLAGERUNGEN IN EINEM KRAFTSTOFF, INSBESONDERE EINES IN FLUGZEUGEN VERWENDETEN KRAFTSTOFFS
DEVICE FOR ANALYSING THE TENDENCY TO THE FORMATION OF DEPOSITS IN A FUEL, IN PARTICULAR A FUEL USED IN AIRCRAFTS

(30) Priorité: 11.02.2016 FR 1651086
(43) Date de publication de la demande: 19.12.2018
(73) Titulaire: IFP Energies nouvelles, 92500 Rueil-Malmaison (FR)
(72) Inventeur: BEN AMARA, Arij, 78230 Le Pecq (FR); ALVES FORTUNATO, Maira, 78420 Carrières-sur-Seine (FR); STARCK, Laurie, 92500 Rueil Malmaison (FR)
(86) Numéro de dépôt international: PCT/EP2017/051929
(87) Numéro de publication internationale: WO 2017/137274

(56) Documents cités:
- GB-A- 2 281 620
- GB-A- 2 292 607
- US-A- 5 337 599
- US-A1- 2012 014 409
- US-A1- 2013 230 926

## Description

La présente invention se rapporte à un appareil d'analyse d'un carburant, notamment de carburants utilisés en aéronautique ou carburéacteur.

Elle concerne plus particulièrement un appareil permettant d'analyser la sensibilité à la formation de dépôt de ce carburéacteur sur une plage étendue de températures et de conditions opératoires (débit carburant, métallurgie, temps de séjour).

La stabilité d'un carburéacteur suscite un intérêt important de la part de l'industrie aéronautique. Cela est dû, d'une part, à l'augmentation des contraintes thermiques et rhéologiques des nouveaux systèmes de combustion et, d'autre part, à la diversification des carburants et des procédés utilisés.

Les carburéacteurs alternatifs bénéficient en outre d'une attention toute particulière comme étant des vecteurs de diversité énergétique et de réduction de l'impact environnemental du transport aérien.

Ces nouveaux carburéacteurs impliquent toutefois des propriétés physico-chimiques nouvelles qui peuvent impacter la stabilité thermo-oxydative de ces carburéacteurs.

Une méthode de qualification de la stabilité thermique des carburéacteurs intitulé "Jet Fuel Thermal Oxidation Tester" (JFTOT) et dont un exemple est illustré par le brevet US 5 293 218, consiste à faire circuler un carburéacteur autour d'un tube chauffé.

Cette méthode permet de qualifier les carburéacteurs selon deux paramètres:
- la formation de particules dans le fluide mesurée par l'écart de pression à travers un filtre (DP filter) et
- l'évaluation de dépôts solides formés sur le tube d'essai, faite par une évaluation visuelle ou quantitative.

Le test est fait à 260°C sur les carburéacteurs conventionnels, à 325°C pour les carburéacteurs alternatifs de type SPK (Synthetic Paraffinic Kerosene) et à 355°C pour les carburéacteurs de type SIP (Synthetized Iso Paraffin). Ces températures sont définies au sein du processus de certification ASTM par les acteurs du domaine (pétroliers, motoristes...).

Cette diversité des températures de certification montre bien le besoin de connaître la sensibilité d'un carburéacteur à la formation de dépôt en fonction de la température.

Les détails de cette procédure sont mieux décrits dans la méthode standard ASTM D3241-14be1 (Standard Test Method for Thermal Oxidation Stability of Aviation Turbine Fuels, 2014).

D'autres méthodes de qualification de la stabilité thermique sont décrites dans les demandes de brevet US 5,337,599 et US 2012/0014409.

En outre, la demande de brevet GB 2292607 décrit un système de mesure de dépôts d'hydrocarbures dans le domaine du raffinage.

Il a été constaté que la température est un facteur clé dans la dégradation thermo-oxydative des carburants, la formation de dépôt et le type de dépôt ainsi formé.

On distingue généralement, dans ce cadre, le régime d'autoxydation, généralement entre 100 et 350°C, le régime pyrolytique qui implique des réactions de craquage et de polymérisation thermique ou catalytique, au-delà de 400°C, et une phase de transition qui fait intervenir les deux régimes simultanément.

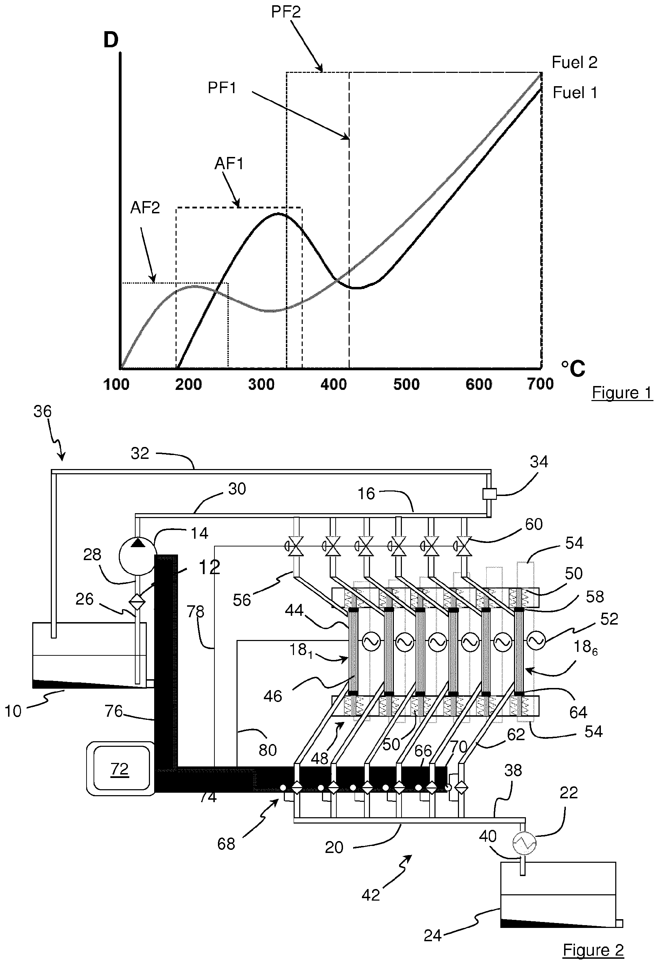

La Figure 1 présente un profil type de la formation de dépôt (D) en fonction de la température (°C) de deux carburéacteurs Fuel 1 et Fuel 2. Le profil Fuel 1 est un profil typique d'un carburéacteur conventionnel et le profil Fuel 2 est la représentation d'un profil type de carburéacteur alternatif HEFA-SPK pour « Hydrotreated Esters and Fatty Acids - Synthetic Paraffinic Kerosine ».

Comme visible sur cette figure, le carburéacteur conventionnel Fuel 1 présente un régime d'autoxydation entre 200 et 300°C et un régime pyrolytique à partir de 425°C alors que le carburéacteur alternatif Fuel 2 présente un régime d'autoxydation entre 100 et 250°C et un régime pyrolytique à partir de 325°C.

Par rapport au circuit que va emprunter le carburéacteur t dans l'aéronef, ce qui va être important de maitriser est sa sensibilité à former du dépôt aux températures basses et moyennes donc en dessous d'environ 400°C. Ainsi, pour le carburateur Fuel 1 le niveau de dépôt croit entre environ 200 et 300°C où il atteint un maximum. Le test de la spécification fait à 260°C est favorablement représentatif du régime où la quantité de dépôt augmente avec la température. Tandis que pour le carburéacteur Fuel 2, le niveau de dépôt augmente à plus basse température. Il atteint un maximum à environ 200°C. Ainsi, la mesure de la spécification réalisée à 325°C ne permet pas de représenter la formation de dépôt à des régimes de températures inférieurs.

Ainsi, il peut être constaté que la composition chimique du carburéacteur impacte ces régimes de dégradation thermo-oxydative et, par conséquence, la morphologie du dépôt et ses caractéristiques. De ce fait, l'évaluation de la stabilité thermique faite à une température unique, semble limitée voire obsolète.

Pour remédier à cela, il est nécessaire d'avoir une évaluation du comportement du carburéacteur sur une plage de température plus large, et ainsi, plus représentative de conditions réelles.

La présente invention concerne donc un appareil d'analyse à la sensibilité à la formation de dépôt dans un carburant, notamment dans un carburant utilisé en aéronautique, comprenant un réservoir de carburant à analyser, un filtre pour le carburant sortant du réservoir, et une pompe de circulation du carburant, au moins deux sections de test identiques placées en parallèle, chaque section de test comprenant un tube chauffé sur lequel circule le carburant et un filtre associé à système de mesure du dépôt. Cet appareil comprend une unité de contrôle gérant indépendamment au moins une des conditions opératoires d'au moins d'une des deux sections de test. Chaque section de test comprend un conduit rigide vertical à l'intérieur duquel est logé le tube métallique de moindre diamètre, une tubulure d'amenée de carburant entre un rail de distribution et l'extrémité supérieure du conduit, un système de contrôle de débit placé sur la tubulure, une tubulure d'évacuation du carburant reliant l'extrémité inférieure du conduit avec un rail d'évacuation et portant ledit filtre associé audit système de mesure du dépôt.

L'une des conditions opératoires peut être la variation de la température du tube chauffé.

L'une des conditions opératoires peut être la variation du débit du carburant circulant sur le tube chauffé.

L'une des conditions opératoires peut être la variation de la durée de l'analyse de la section de test.

Le carburant peut être un carburéacteur, un carburant Diesel ou un carburant Essence.

L'invention concerne également un procédé d'analyse à la sensibilité à la formation de dépôt dans un carburant, notamment dans un carburant utilisé en aéronautique, dans lequel on fait circuler ledit carburant à analyser en utilisant une pompe de circulation depuis un réservoir vers un filtre pour le carburant et au moins deux sections de test identiques placées en parallèle, chaque section de test comprenant un tube chauffé et un filtre associé à un système de mesure du dépôt, un conduit rigide vertical à l'intérieur duquel est logé un tube métallique de moindre diamètre, une tubulure d'amenée de carburant entre un rail de distribution et l'extrémité supérieure du conduit, un système de contrôle de débit placé sur la tubulure, une tubulure d'évacuation du carburant reliant l'extrémité inférieure du conduit avec un rail d'évacuation et portant ledit filtre associé audit système de mesure du dépôt. Selon ce procédé on il y a une étape de gestion indépendante d'au moins une des conditions opératoires d'au moins d'une des deux sections de test.

Les autres caractéristiques et avantages de l'invention vont apparaître maintenant à la lecture de la description qui va suivre, donnée à titre uniquement illustratif et non limitatif, et à laquelle sont annexées, outre la figure 1 décrite précédemment, la figure 2 qui illustre un appareil d'analyse d'un carburant selon un mode de réalisation de l'invention, notamment d'un carburant utilisé en aéronautique ou carburéacteur.

Sur la figure 2, l'appareil d'analyse est un appareil, selon un mode de réalisation de l'invention, avec un système de circulation dynamique de carburant à analyser, ici un carburéacteur conventionnel ou alternatif.

Cet appareil comprend un réservoir d'alimentation 10 contenant le carburéacteur, un filtre 12 pour le carburéacteur sortant du réservoir et une pompe de circulation 14 de ce carburéacteur. Ce circuit comprend également un rail de distribution 16 alimentant en carburéacteur pompé au moins deux sections de test, ici six sections 18₁ à 18₆, placées en parallèle les unes aux autres et un rail d'évacuation 20 du carburéacteur qui a traversé les sections de test.

Avantageusement, ce rail d'évacuation est relié à un échangeur de chaleur 22 placé en aval d'un réservoir de récupération 24 de carburéacteur usagé.

Ce circuit comprend en outre différentes conduites de circulation du carburéacteur pour relier les éléments de ce circuit.

Ainsi, une conduite 26 relie le réservoir 10 au filtre 12, une autre conduite 28 relie le filtre à la pompe 14, encore une autre conduite 30 relie la pompe avec le rail de distribution 16 et enfin une conduite de retour 32 relie le rail de distribution et le réservoir d'alimentation, cette conduite portant un régulateur de débit 34.

Tous ces éléments forment ainsi un circuit d'alimentation 36 pour les sections de test.

Comme mieux visible sur la figure 2, le rail d'évacuation 20 est relié par une conduite 38 à l'échangeur 22 qui est lui-même relié par une conduite 40 au réservoir de récupération en formant un circuit d'évacuation 42 de carburant pour les sections de test.

Chaque section de test comprend un conduit rigide vertical 44 à l'intérieur duquel est logé un tube métallique 46 de moindre diamètre, des moyens de chauffage 48, ici sous la forme de résistances électriques 50 placées à chaque extrémité du tube, pour chauffer ce tube à la même température, une alimentation électrique 52 de ces résistances par des conducteurs 54, une tubulure d'amenée 56 de carburéacteur entre le rail de distribution 16 et l'extrémité supérieure 58 du conduit 44, un système de contrôle de débit 60 placé sur la tubulure, une tubulure d'évacuation 62 du carburéacteur reliant l'extrémité inférieure 64 du conduit 44 avec le rail d'évacuation 20 et portant un filtre 66 du carburéacteur, et un système de mesure 68 du dépôt provenant du carburéacteur déposé sur le filtre.

De manière préférentielle, ce système de mesure comprend un capteur de pression différentielle 70 qui mesure le différentiel de pression entre l'amont et l'aval de ce filtre.

Cet appareil comprend également une unité de contrôle 72 qui gère indépendamment au moins une des conditions opératoires d'au moins d'une des deux sections de test, notamment la température des tubes, ainsi que la réalisation en automatique de l'analyse, et qui enregistre toutes les acquisitions en temps réel.

Plus particulièrement, cette unité est reliée par une ligne de contrôle 74 au capteur différentiel 70 pour connaitre l'état de colmatage du filtre, par une ligne de commande 76 à la pompe de circulation 14, par une autre ligne de commande 78 au système de contrôle de débit 60 et encore par une autre ligne de commande 80 à l'alimentation électrique 52 des résistances.

En fonctionnement, l'unité de contrôle 72 commande indépendamment au moins un, de préférence chaque, moyen de chauffage 48 du tube 46. La température de chauffage de chaque tube peut être régulée séparément entre 100 et 400°C pour chaque section de test. La pompe 14 extrait le carburéacteur du réservoir 10 pour le faire passer au travers du filtre 12. A la sortie de la pompe, le carburéacteur est amené au rail de distribution 16 pour alimenter toutes les tubulures d'amenée 56 des sections de test 18₁ à 18₆. Le débit du carburéacteur circulant dans chaque tubulure 56 est contrôlé de manière indépendante par le système de contrôle de débit 60 gérés par l'unité de contrôle 72.

A partir de la tubulure, le carburéacteur pénètre dans le conduit rigide 44 et circule tout au long de la paroi externe du tube 46. Sous l'effet de la chaleur de ce tube, il se forme des dépôts provenant de ce carburéacteur dont une partie se dépose sur cette paroi externe et une autre partie est mélangée avec le carburéacteur.

Le carburéacteur et les dépôts dissous qu'il contient sont évacués par la tubulure d'évacuation 62 vers le réservoir de récupération 24.

Durant cette évacuation, le carburéacteur traverse le filtre 66 qui retient les dépôts que contient ce carburéacteur, puis ce carburéacteur est refroidi aux environs de 20°C par l'échangeur 22 avant de pénétrer dans le réservoir de récupération.

Le carburéacteur en excès au niveau du rail de distribution 16 est retourné au réservoir d'alimentation 10 par la conduite 32.

L'analyse est arrêtée après une durée déterminée, cette durée peut être définie séparément pour chaque section de test.

Il est à noter que le différentiel de pression à travers chaque filtres 66 est suivi en temps réel par le capteur de pression différentielle associé 70. Ce dernier indique la formation de produits de dégradation dans le carburant liquide.

De plus, les dépôts solides formés sur les tubes peuvent être caractérisés en terme d'épaisseur et de volume, soit ex-situ, par une méthode de quantification, par exemple, l'interférométrie ou l'ellipsométrie décrites dans la norme ASTM D 3241, soit in-situ, par l'utilisation de conduits rigides transparents permettant la traversée de faisceau lumineux couplé à une méthode optique, par exemple, l'interférométrie ou l'ellipsométrie décrites dans la norme ASTM D3241.

Comme illustré par l'exemple qui suit, l'appareil décrit plus haut permet de gérer de manière automatique la réalisation de l'essai. Ce dernier consiste à reproduire en condition dynamique des dépôts sur plusieurs sections disposées en parallèle, soumises à des conditions opératoires différentes.

La caractérisation du carburant inclut toutes ou une parties des variations suivantes :
1) Variation de la température: Le test est réalisé à plusieurs températures sur chaque section de test. La température Ti recommandée est entre 160 et 360°C. La variation se fait de préférence sur au moins cinq sections de test différentes, toutes conditions égales par ailleurs : Durée Di (durée recommandée 2.5h), état de surface Wi (Wi recommandé compatible avec ASTM D 3241), débit carburant ϕi (ϕi recommandé 3mL/min).
2) Variation du débit de carburant : Le débit recommandé est entre 0 et 30 mL/min. La variation se fait de préférence sur au moins deux sections de test différentes, toutes conditions égales par ailleurs : Température Ti (Ti recommandée 260°C), Durée Di (Di recommandée 2.5h), état de surface Wi (Wi recommandé compatible avec ASTM D 3241)
3) Variation de la durée d'essai : La durée recommandée *Di* varie entre 0 et 30 heures La variation se fait de préférence sur au moins deux sections différentes, toutes conditions égales par ailleurs : Température Ti (Ti recommandée 260°C), débit carburant ϕi (ϕi recommandé 3mL/min) et état de surface Wi (Wi recommandé compatible avec ASTM D 3241),
4) Variation des caractéristiques du tube : Des tubes non-standards peuvent être évalués. Ces derniers peuvent présenter des types de matériaux, des revêtements ou des états de surface différents. Par exemple, par l'utilisation de matériaux ayant une fonction catalytique comme le cuivre, le zinc et le fer. La variation se fait de préférence sur au moins deux sections différentes, toutes conditions égales par ailleurs : Température Ti (Ti recommandée 260°C), débit carburant ϕi (ϕi recommandé 3mL/min) et Durée Di (Di recommandée 2.5h).

A la fin de chaque analyse, le dépôt formé sur les tubes 46 est caractérisé par une méthode de quantification (par exemple les méthodes ASTM D3241).

Avantageusement, un module d'analyse permet de regrouper les résultats de toutes les sections. Le module construit alors les courbes de variation de la quantité de dépôts en fonction des paramètres évalués, à savoir, la température, la durée de l'analyse, la vitesse d'écoulement et les caractéristiques de la surface. Ces différentes courbes permettent de cartographier intégralement la stabilité thermique du carburéacteur.

Ainsi, cette cartographie constitue :
- Un moyen d'analyse de l'effet des paramètres clé sur la formation de dépôt.
- Un outil prédictif qui permet :
   ∘ 1) d'estimer la stabilité d'un carburéacteur sur toutes les températures intermédiaires non testées et
   ∘ 2) d'estimer la température de rupture (breakpoint).
- Un outil préventif pour alerter l'utilisateur pour tout risque de défaillance en associant les données cartographiées avec les dimensions et les conditions opératoires de l'utilisateur

La présente invention n'est pas limitée à l'exemple décrit ci-dessus mais peut s'appliquer à tous types de carburants autre qu'aux carburéacteurs.

En particulier, le carburant analysé peut être un diesel, un biodiesel, ou une essence.

## Revendications

1. Appareil d'analyse de la sensibilité à la formation de dépôt dans un carburant, notamment dans un carburant utilisé en aéronautique, comprenant un réservoir (10) de carburant à analyser, un filtre (12) pour le carburant sortant du réservoir, et une pompe de circulation (14) du carburant, au moins deux sections de test (18), chaque section de test comprenant un tube métallique (46) chauffé sur lequel circule le carburant et un filtre (66) associé à système de mesure du dépôt (68), ledit appareil comprenant aussi au moins deux sections de test (18₁ à 18₆) identiques placées en parallèle et une unité de contrôle (72) adaptée pour gérer indépendamment au moins une des conditions opératoires d'au moins d'une des deux sections de test, et où chaque section de test (18) comprend un conduit rigide vertical (44) à l'intérieur duquel est logé ledit tube métallique (46) de moindre diamètre, une tubulure d'amenée (56) de carburant entre un rail de distribution (16) et l'extrémité supérieure (58) du conduit (44), un système de contrôle de débit (60) placé sur la tubulure (56), une tubulure d'évacuation (62) du carburant reliant l'extrémité inférieure (64) du conduit (44) avec un rail d'évacuation (20) et portant ledit filtre (66) associé audit système de mesure du dépôt (68) .

2. Appareil d'analyse selon la revendication 1, **caractérisé en ce que** l'une des conditions opératoires est la variation de la température (Ti) du tube (46) chauffé.

3. Appareil d'analyse selon la revendication 1 ou 2, **caractérisé en ce que** l'une des conditions opératoires est la variation du débit (ϕi) du carburant circulant sur le tube (46) chauffé.

4. Appareil d'analyse selon l'une des revendications précédentes, **caractérisé en ce que** l'une des conditions opératoires est la variation de la durée (Di) de l'analyse de la section de test.

5. Appareil d'analyse selon l'une des revendications précédentes, **caractérisé en ce que** le système de mesure du dépôt (68) comprend un capteur de pression différentielle (70).

6. Procédé d'analyse de la sensibilité à la formation de dépôt dans un carburant, notamment dans un carburant utilisé en aéronautique, dans lequel on fait circuler ledit carburant à analyser en utilisant une pompe de circulation (14) depuis un réservoir (10) vers un filtre (12) pour le carburant et au moins deux sections de test (18), chaque section de test comprenant un tube (46) chauffé et un filtre (66) associé à un système de mesure du dépôt (68), un conduit rigide vertical (44) à l'intérieur duquel est logé un tube métallique (46) de moindre diamètre, une tubulure d'amenée (56) de carburant entre un rail de distribution (16) et l'extrémité supérieure (58) du conduit (44), un système de contrôle de débit (60) placé sur la tubulure (56), une tubulure d'évacuation (62) du carburant reliant l'extrémité inférieure (64) du conduit (44) avec un rail d'évacuation (20) et portant ledit filtre (66) associé audit système de mesure du dépôt (68), ledit procédé comprenant aussi qu'on fait circuler ledit carburant à analyser dans au moins deux sections de test identiques placées en parallèle, et comprenant une étape de gestion indépendante d'au moins une des conditions opératoires d'au moins d'une des deux sections de test.

7. Procédé d'analyse selon la revendication 6, **caractérisé en ce que** le carburant est un carburéacteur.

8. Procédé d'analyse selon la revendication 6, **caractérisé en ce que** le carburant est un carburant Diesel.

9. Procédé d'analyse selon la revendication 6, **caractérisé en ce que** le carburant est un carburant Essence.

## Patentansprüche

1. Vorrichtung zur Analyse der Empfindlichkeit für die Ablagerungsbildung in einem Kraftstoff, insbesondere einem Kraftstoff, der in der Luftfahrt genutzt wird, umfassend einen Vorratsbehälter (10) für zu analysierenden Kraftstoff, einen Filter (12) für den Kraftstoff, der aus dem Vorratsbehälter austritt, und eine Pumpe zur Zirkulation (14) des Kraftstoffs, mindestens zwei Prüfabschnitte (18), wobei jeder Prüfabschnitt ein erhitztes Metallrohr (46) umfasst, in dem der Kraftstoff zirkuliert, und einen Filter (66), der einem System zur Messung der Ablagerung (68) zugeordnet ist, die Vorrichtung ebenfalls umfassend mindestens zwei identische Prüfabschnitte (18₁ bis 18₆), die parallel angeordnet sind, und eine Steuereinheit (72), die geeignet ist, mindestens eine der Betriebsbedingungen mindestens einer der zwei Prüfabschnitte unabhängig zu verwalten, und wobei jeder Prüfabschnitt (18) eine starre vertikale Leitung (44), innerhalb welcher das Metallrohr (46) geringeren Durchmessers untergebracht ist, umfasst, einen Kraftstoffzulaufstutzen (56) zwischen einer Verteilerschiene (16) und dem oberen Ende (58) der Leitung (44), ein Durchsatzsteuersystem (60), das an dem Stutzen (56) platziert ist, einen Stutzen zum Ableiten (62) des Kraftstoffs, der das untere Ende (64) der Leitung (44) mit einer Ableitschiene (20) verbindet und den Filter (66) trägt, der dem System zur Messung der Ablagerung (68) zugeordnet ist.

2. Analysevorrichtung nach Anspruch 1, **dadurch gekennzeichnet, dass** eine der Betriebsbedingungen die Änderung der Temperatur (Ti) des erhitzten Rohrs (46) ist.

3. Analysevorrichtung nach Anspruch 1 oder 2, **dadurch gekennzeichnet, dass** eine der Betriebsbedingungen die Änderung des Durchsatzes (ϕi) des Kraftstoffs ist, der im erhitzten Rohr (46) zirkuliert.

4. Analysevorrichtung nach einem der vorhergehenden Ansprüche, **dadurch gekennzeichnet, dass** eine der Betriebsbedingungen die Änderung der Dauer (Di) der Analyse des Prüfabschnitts ist.

5. Analysevorrichtung nach einem der vorhergehenden Ansprüche, **dadurch gekennzeichnet, dass** das System zur Messung der Ablagerung (68) einen Differenzdrucksensor (70) umfasst.

6. Verfahren zur Analyse der Empfindlichkeit für die Ablagerungsbildung in einem Kraftstoff, insbesondere in einem Kraftstoff, der in der Luftfahrt genutzt wird, wobei der zu analysierende Kraftstoff unter Nutzung einer Zirkulationspumpe (14) von einem Vorratsbehälter (10) zu einem Filter (12) für den Kraftstoff und mindestens zwei Prüfabschnitte (18) zirkulieren gelassen wird, wobei jeder Prüfabschnitt ein erhitztes Rohr (46) und einen Filter (66), der einem System zur Messung der Ablagerung (68) zugeordnet ist, eine starre vertikale Leitung (44), innerhalb welcher ein Metallrohr (46) geringeren Durchmessers untergebracht ist, einen Kraftstoffzulaufstutzen (56) zwischen einer Verteilerschiene (16) und dem oberen Ende (58) der Leitung (44), ein Durchsatzsteuersystem (60), das an dem Stutzen (56) platziert ist, einen Stutzen zum Ableiten (62) des Kraftstoffs, der das untere Ende (64) der Leitung (44) mit einer Ableitschiene (20) verbindet und den Filter (66) trägt, der dem System zur Messung der Ablagerung (68) zugeordnet ist, umfasst, wobei das Verfahren ebenfalls umfasst, dass der zu analysierende Kraftstoff in mindestens zwei identischen Prüfabschnitten zirkulieren gelassen wird, die parallel angeordnet sind, und einen Schritt des unabhängigen Verwaltens mindestens einer der Betriebsbedingungen mindestens eines der zwei Prüfabschnitte umfasst.

7. Analyseverfahren nach Anspruch 6, **dadurch gekennzeichnet, dass** der Kraftstoff ein Düsentreibstoff ist.

8. Analyseverfahren nach Anspruch 6, **dadurch gekennzeichnet, dass** der Kraftstoff ein Dieselkraftstoff ist.

9. Analyseverfahren nach Anspruch 6, **dadurch gekennzeichnet, dass** der Kraftstoff ein Benzinkraftstoff ist.

## Claims

1. Analysis device for analysing the tendency to the formation of deposits in a fuel, particularly in a fuel used in aviation, comprising a tank (10) of fuel to be analysed, a filter (12) for the fuel leaving the tank, and a fuel circulation pump (14), at least two test sections (18), each test section comprising a heated metal tube (46) over which the fuel circulates and a filter (66) associated with a deposit measurement system (68), said device also comprising at least two identical test sections (18₁ to 18₆) placed in parallel, and a control unit (72) designed to manage, independently, at least one of the operating conditions of at least one of the two test sections, and wherein each test section (18) comprises a vertical rigid duct (44) inside which said smaller-diameter metal tube (46) is housed, a fuel-conveying pipe (56) conveying fuel between a distribution rail (16) and the upper end (58) of the duct (44), a flow rate control system (60) placed on the pipe (56), a fuel discharge pipe (62) connecting the lower end (64) of the duct (44) to a discharge rail (20) and supporting said filter (66) associated with said deposit measurement system (68) .

2. Analysis device according to Claim 1, **characterized in that** one of the operating conditions is the variation in the temperature (Ti) of the heated tube (46) .

3. Analysis device according to Claim 1 or 2, **characterized in that** one of the operating conditions is the variation in the flow rate (ϕi) of the fuel circulating over the heated tube (46).

4. Analysis device according to one of the preceding claims, **characterized in that** one of the operating conditions is the variation in the duration (Di) of the analysis of the test section.

5. Analysis device according to one of the preceding claims, **characterized in that** the deposit measurement system (68) comprises a differential-pressure sensor (70).

6. Method for analysing the tendency to the formation of deposits in a fuel, particularly in a fuel used in aviation, wherein said fuel that is to be analysed is circulated using a circulation pump (14) from a tank (10) towards a filter (12) for the fuel and at least two test sections (18), each test section comprising a heated tube (46) and a filter (66) associated with a deposit measurement system (68), a vertical rigid duct (44) inside which is housed a smaller-diameter metal tube (46), a fuel-conveying pipe (56) conveying fuel between a distribution rail (16) and the upper end (58) of the duct (44), a flow rate control system (60) placed on the pipe (56), a fuel discharge pipe (62) connecting the lower end (64) of the duct (44) to a discharge rail (20) and supporting said filter (66) associated with said deposit measurement system (68), said method also involving that said fuel that is to be analysed is made to circulate in at least two identical test sections placed in parallel and comprising a step of independent management of at least one of the operating conditions of at least one of the two test sections.

7. Analysis method according to Claim 6, **characterized in that** the fuel is a jet fuel.

8. Analysis method according to Claim 6, **characterized in that** the fuel is a diesel fuel.

9. Analysis method according to Claim 6, **characterized in that** the fuel is a gasoline fuel.
